(19) Europäisches Patentamt
European Patent Office
Office européen des brevets

(11) **EP 4 717 120 A1**

(12) **EUROPEAN PATENT APPLICATION**

(43) Date of publication:
**01.04.2026 Bulletin 2026/14**

(21) Application number: **24203495.7**

(22) Date of filing: **30.09.2024**

(51) International Patent Classification (IPC):
**A46B 15/00** (2006.01)    **A61B 5/00** (2006.01)

(52) Cooperative Patent Classification (CPC):
**A46B 15/0006; A46B 15/0008; A61B 5/6843;**
**A61B 5/6885;** A46B 2200/1066; A61B 2560/0233;
A61B 2562/0233; A61B 2562/0247

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **VAN NOORD, Kris**
**Eindhoven (NL)**
• **MULDER, Bernardo Arnoldus**
**Eindhoven (NL)**

(74) Representative: **Philips Intellectual Property &**
**Standards**
**High Tech Campus 52**
**5656 AG Eindhoven (NL)**

(54) **TEMPERATURE COMPENSATION IN OPTICAL SENSORS FOR ORAL CLEANING DEVICES**

(57)    Disclosed herein is an oral cleaning device (100) comprising: a drive shaft (106) for receiving a cleaning element (118), a reflective element (108) attached to the drive shaft, a mechanical drive (110) for mechanically operating the drive shaft, and an optical sensor (112, 112') configured for measuring a displacement (500, 600) of the drive shaft during operation of the oral cleaning device. The optical sensor comprises at least one light source (114) optically coupled to multiple light sensors (116) via the reflective element to provides optical sensor specific sensor data (138). The execution of the machine executable instructions (128) causes a computational (126) system to: measure (202) the optical sensor specific sensor data if the oral cleaning device is in a stationary state (400), measure (204) the temperature of the optical sensor, determine (206) a sensor specific thermal compensation calibration (140); and store (208) the sensor specific thermal compensation calibration.

Fig. 1

EP 4 717 120 A1

**Description**

TECHNICAL FIELD OF THE INVENTION

The invention relates to oral cleaning devices, in particular to the use of sensors to determine the mechanical load on an oral cleaning device.

BACKGROUND OF THE INVENTION

Oral cleaning devices, such as electric toothbrushes or sonic toothbrushes, are designed to improve dental hygiene by utilizing motor-driven brushing techniques that enhance plaque removal and gum cleaning. These devices often incorporate features like oscillating or sonic movements and timers to ensure thorough and consistent cleaning.

United States patent application publication US 11051609B2 discloses an oral cleaning device for characterizing a position of a brush head of the device. The oral cleaning device includes: a body portion; a brush head extending from the body portion, where at least a portion of the brush head is configured to move relative to the body portion; a controller; and an optical sensor positioned relative to the brush head member and body portion and being in communication with the controller, where the optical sensor is configured to obtain optical sensor data resulting from a deflection or rotation of the brush head member relative to the body portion; the controller being configured to receive the optical sensor data and determine the deflection or rotation of the brush head member relative to the body portion.

SUMMARY OF THE INVENTION

The invention provides for an oral cleaning device, an oral cleaning system, a method of operating an oral cleaning device, and a computer program. Embodiments are given in the dependent claims.

In one aspect an oral cleaning device is disclosed. The oral cleaning device comprises a body portion, a stationary state detection system, a drive shaft configured for receiving a cleaning element, a reflective element attached to the drive shaft, a mechanical drive for mechanically operating the drive shaft, and an optical sensor. The optical sensor is configured for measuring a displacement of the drive shaft during operation of the oral cleaning device. The optical sensor comprises at least one light source and multiple light sensors. The optical sensor has a fixed position relative to the body portion. The at least one light source is optically coupled to the multiple light sensors via the reflective element. The optical sensor provides an optical sensor-specific sensor data.

The oral cleaning device further comprises a temperature sensor configured for measuring temperature data descriptive of a temperature of the optical sensor. The oral cleaning device further comprises a memory storing machine-executable instructions and a mechanical load calibration. The oral cleaning device further comprises a computational system. Execution of the machine-executable instructions causes the computational system to detect if the oral cleaning device is in the stationary state using the stationary state detection system. Execution of the machine-executable instructions further causes the computational system to measure the optical sensor-specific sensor data if the oral cleaning device is in the stationary state. Execution of the machine-executable instructions further causes the computational system to measure the temperature data using the temperature sensor. The temperature data is correlated to the optical sensor-specific sensor data. Execution of the machine-executable instructions further causes the computational system to determine a sensor-specific thermal compensation calibration. Execution of the machine-executable instructions further causes the computational system to store the sensor-specific thermal compensation calibration in the memory.

In another aspect an oral cleaning system is disclosed. The oral cleaning system comprises an example of the oral cleaning device and an external charging unit. In another aspect, a method of operating an oral cleaning device is disclosed. The oral cleaning device comprises a body portion, a stationary state detection system, a drive shaft configured for receiving a cleaning element, a reflective element attached to the drive shaft, a mechanical drive for mechanically operating the drive shaft, and an optical sensor configured for measuring a displacement of the drive shaft during operation of the oral cleaning device. The optical sensor comprises at least one light source and multiple light sensors. The optical sensor has a fixed position relative to the body portion. The at least one light source is optically coupled to the multiple light sensors via the reflective element. The optical sensors provide optical sensor-specific sensor data.

The oral cleaning device further comprises a temperature sensor that is configured for measuring temperature data descriptive of a temperature of the optical sensor. The method comprises detecting if the oral cleaning device is in a stationary state using the stationary state detection system. The method further comprises measuring the optical sensor-specific sensor data if the oral cleaning device is in the stationary state. The method further comprises measuring the temperature data. The temperature data is correlated to the optical sensor-specific sensor data. The method further comprises determining a sensor-specific thermal compensation calibration. The method further comprises storing the sensor-specific thermal compensation calibration in the memory.

In another example, a computer program is disclosed. The computer program comprises machine-executable instructions. The machine-executable instructions are configured to cause a computational system to perform an example of the method.

BRIEF DESCRIPTION OF THE DRAWINGS

In the following preferred embodiments of the invention will be described, by way of example only, and with reference to the drawings in which:

Fig. 1 illustrates an example of an oral cleaning device.
Fig. 2 shows a flow chart which illustrates a method of using the oral cleaning device of Fig. 1.
Fig. 3 shows a flow chart which illustrates a further method of using the oral cleaning device of Fig. 1.
Fig. 4 shows a top view of an optical sensor for measuring a mechanical load on a drive shaft or cleaning element of the oral cleaning device.
Fig. 5 shows the top view of the optical sensor of Fig. 4 with mechanical changes caused by an applied brushing force exerted on the cleaning element.
Fig. 6 shows the top view of the optical sensor of Fig. 4 with mechanical changes caused by a torque applied to the cleaning element.
Fig. 7 shows a graph that illustrates differences in the thermal calibration of different optical sensors.
Fig. 8 shows a plot which illustrates the benefit of using the sensor specific thermal compensation calibration.

DESCRIPTION OF EMBODIMENTS

Like numbered elements in these figures are either equivalent elements or perform the same function. Elements which have been discussed previously will not necessarily be discussed in later figures if the function is equivalent.

In an example there is an oral cleaning device. The oral cleaning device comprises a body portion. The body portion may encompass the large structural elements of the oral cleaning device such as a handle or body of the oral cleaning device. The oral cleaning device further comprises a stationary state detection system. The stationary state detection system is configured to determine if the body portion is in a steady state or not being moved. This may be accomplished in different ways in different examples. In some examples there may be a motion sensor detector such as an accelerometer. In other examples, the oral cleaning device may be able to detect if the oral cleaning device has been placed onto an external charging unit.

The oral cleaning device further comprises a drive shaft configured for receiving a cleaning element. For example, the drive shaft may be a rigid body which is configured for receiving the cleaning element. The cleaning element may be an element which is attached to the oral cleaning device and may be used for performing oral cleaning. An example of a cleaning element would be a toothbrush. The oral cleaning device further comprises a reflective element attached to the drive shaft. The reflective element is able to reflect light that is directed at the reflective element. The oral cleaning device further comprises a mechanical drive. The mechanical drive is configured for mechanically operating the drive shaft. The operation of the drive shaft causes the cleaning element to be moved in a way which may be useful for performing oral cleaning on a subject. For example, it may have a motion for a sonic toothbrush or a reciprocating motion for an electric toothbrush.

The oral cleaning device further comprises an optical sensor configured for measuring a displacement of the drive shaft during operation of the oral cleaning device. When a subject is using an oral cleaning device the subject may damage the subject's gums or other oral tissue if too much force or torque is placed on the cleaning element. The optical sensor may be used to determine the amount of force or torque exerted by a subject when using the cleaning element. The optical sensor comprises at least one light source and multiple light sensors. The optical sensor has a fixed position relative to the body portion. The at least one light source is optically coupled to the multiple light sensors via the reflective element. The optical sensor provides optical sensor-specific sensor data. The optical sensor is able to detect a displacement and thereby the force or torque on the oral cleaning device by detecting a change in the amount of light hitting the multiple light sensors.

The oral cleaning device further comprises a temperature sensor that is configured for measuring temperature data descriptive of a temperature of the optical sensor. The optical sensor is comprised of the at least one light source and the multiple light sensors. The at least one light source and the multiple light sensors may both have a temperature dependency. A transistor or transistors, which may also have a temperature dependence, may also be used to control the output of the at least one light source. By monitoring the temperature with the temperature sensor it may enable the compensation of the optical sensor for changes in temperature.

The oral cleaning device further comprises a memory storing machine-executable instructions and a mechanical load calibration. The mechanical load calibration may for example contain sensor data which calibrates the change in the optical sensor data to infer the force or torque. The oral cleaning device further comprises a computational system. The computational system may for example encompass a processor, a microcontroller, FPGA or other device which is able to perform basic control tasks and perform calculations. Execution of the machine-executable instructions causes the computational system to detect if the oral cleaning device is in a stationary state using the stationary state detection system. In the stationary state the oral cleaning device may for example be not in use and being left in a single position. This may mean that the torque or force exerted on the drive shaft may be constant or be minimized.

Execution of the machine-executable instructions further causes the computational system to measure the optical sensor-specific sensor data if the oral cleaning device is in the stationary state. Execution of the machine-executable instructions further causes the computational system to measure the temperature data using the temperature sensor. The temperature data is correlated to the optical sensor-specific sensor data. This may mean that the optical sensor-specific sensor data is assigned to or registered to the temperature data, so for the corresponding temperature the optical sensor-specific sensor data when the oral cleaning device is in the stationary state is known.

Execution of the machine-executable instructions further causes the computational system to determine a sensor-specific thermal compensation calibration. The change in the at least one light source and the multiple light sensors may be an offset measured by the multiple light sensors as a function of temperature. The sensor-specific thermal compensation calibration may provide then a value which can be added or subtracted to the optical sensor-specific sensor data to correct for temperature.

The measurement of the optical sensor-specific sensor data and the temperature data may be performed more than once in different examples. This may provide for a more accurate sensor-specific thermal compensation calibration. It may for example enable the averaging of data points as well as providing a linear or curved fit model of the sensor-specific thermal compensation calibration. Execution of the machine-executable instructions further causes the computational system to store the sensor-specific thermal compensation calibration in the memory. This may for example enable the sensor-specific thermal compensation calibration to be used when the oral cleaning device is in operation and the mechanical drive is mechanically operating the drive shaft to actuate or move the cleaning element. This may for example provide for improved determination of the force or torque which is exerted on the cleaning element.

In another example, execution of the machine-executable instructions further causes the computational system to regulate operation of the mechanical drive using the optical sensor-specific sensor data and the sensor-specific thermal compensation calibration. The sensor-specific thermal compensation calibration can be used to make measurements made with the optical sensor-specific sensor data more accurate. This may for example be used to control the mechanical drive when too much force or torque is being exerted on the cleaning element. In another example, the operation of the mechanical drive may be regulated by flashing a warning light or symbol to an operator indicating for them to turn off the oral cleaning device or to adjust use of it. This may for example provide for an oral cleaning device which not only cleans the subject's oral cavity or teeth or gums more accurately, but also may provide for a higher degree of safety.

In another example, execution of the machine-executable instructions further causes the computational system to measure the temperature data using the sensor data during the operation of the mechanical drive. Execution of the machine-executable instructions further causes the computational system to measure the optical sensor-specific sensor data during the operation of the mechanical drive. Execution of the machine-executable instructions further causes the computational system to determine corrected optical sensor-specific sensor data using the measured optical sensor-specific sensor data, the temperature data, and the sensor-specific thermal compensation calibration. For example, the temperature data and the sensor-specific thermal compensation calibration may be used to determine an offset which is used to correct the measured optical sensor-specific sensor data.

Execution of the machine-executable instructions further causes the computational system to determine a temperature-corrected mechanical load on the drive shaft using the corrected optical sensor-specific sensor data. As was mentioned above, the offset to the measured optical sensor-specific sensor data may be compensated for to obtain the temperature-corrected mechanical load. Execution of the machine-executable instructions further causes the computational system to provide a warning signal if the mechanical load is outside of a predetermined mechanical load range. For example, if the mechanical load is too large or might be in a range which would injure the subject, the warning signal can be provided. This may for example be an optical or auditory signal, it may also be a signal that is transmitted to a personal computing device such as a Bluetooth warning. In another example, the warning signal may be used to regulate or control the operation of the mechanical drive. For example, if the mechanical load is above a predetermined threshold then it may cause the operation of the mechanical drive to be shut off

In another example, the temperature-corrected load is an external force determined by a reduction in the magnitude of the corrected optical sensor-specific sensor data being proportional for the multiple light sensors. For example, it was mentioned above that the at least one light source is coupled into the multiple light sensors by the reflective element. As the drive shaft and also the reflective element are moved, then this may result in the reduction in the magnitude to the multiple light sensors as a whole. This must be used in conjunction with the mechanical load calibration to determine the force on the cleaning element or the drive shaft.

In another example, the temperature-corrected mechanical load is an external torque determined by a change in the magnitude of the corrected optical sensor-specific sensor data calculated as a ratio between the multiple light sensors. If the reflective element is twisted due to a torque, this may cause an increase in one of the at least one optical sensors and a decrease in the signal from at least one of the optical sensors. By looking at this ratio and using the mechanical load calibration the torque may be determined.

In another example, the determination of the temperature-corrected mechanical load is repeatedly updated during continual operation of the oral cleaning device by repeatedly measuring the temperature data and by repeatedly measuring the optical sensor-specific sensor data. During heavy use, the mechanical drive may wind up heating components which are in the body portion. For example, the mechanical drive may cause heating which causes an increase in temperature of the at least one light source and/or the multiple light sensors. This may result in an offset in the optical sensor-specific sensor data. By repeatedly measuring the temperature and using the calibration this offset may be compensated for. This may result in more accurate determination of the mechanical load such as the external force or torque that is applied to the cleaning element.

In another example, the sensor-specific thermal compensation calibration is any one of the following: a sensor-specific linear regression calibration, a sensor-specific two-point linear fit, a sensor-specific spline fit, and a sensor-specific interpolation between calibration points. The offset as a function of temperature is to a large degree linear. The two-point linear fit for two calibration values may therefore result in a valid thermal compensation calibration. However, if additional data points are used and a linear regression is calculated or there is averaging of data points, this may result in a more accurate sensor-specific thermal compensation calibration. Likewise, the use of sensor-specific spline fit or interpolation between calibration points may also assist in more accurately determining the offset in the optical sensor-specific sensor data due to changes in temperature.

In another example, execution of the machine-executable instructions further causes the computational system to determine the sensor-specific thermal compensation calibration by collecting data during multiple instances of being in the stationary state. This may be beneficial because at different times the optical sensor may have a different temperature. For example, if the oral cleaning device experienced heavy use it may be at an elevated temperature or during the summer there may also be elevated temperatures or cooler temperatures during the winter. By taking multiple measurements of the temperature data and the optical sensor-specific sensor data a more accurate sensor-specific thermal compensation calibration can be determined.

In another example, the determining of the sensor-specific thermal compensation calibration over multiple placements of the oral cleaning device comprises any one of the following: revising a fit to previously acquired pairs of the temperature data and the optical sensor-specific sensor data, replacing the previously acquired pairs of temperature data and the optical sensor-specific sensor data, and averaging with the previously acquired pairs of the temperature data and the optical sensor-specific sensor data. All of these options may provide for a means of better determining the sensor-specific thermal compensation calibration.

In another example, execution of the machine-executable instructions further causes the computational system to calculate projected optical sensor-specific sensor data by inputting the temperature data into the sensor-specific thermal compensation calibration. In this step, the temperature can be measured and this can be used to work backwards and estimate what the optical sensor-specific sensor data should be for that particular temperature. Execution of the machine-executable instructions further causes the computational system to perform any one of the following if the optical sensor-specific sensor data differs from the projected optical sensor-specific sensor data by more than a predetermined amount: provide a calibration warning signal, initiate a thermal compensation calibration recalibration procedure, and combinations thereof. If the projected optical sensor-specific sensor data is too far out from what was predicted by the calibration, then this may mean that there is damage to the oral cleaning device and may be needing service or replacement or it could mean that for example, the aging of the optical components has changed their optical properties and it may then initiate a recalibration procedure. This may for example involve deleting all or some of the data used for calculating the sensor-specific thermal compensation calibration or going through and recalculating it completely.

In another example, the oral cleaning device further comprises a heater configured for heating the optical sensor. Execution of the machine-executable instructions further causes the computational system to control the heater to heat the optical sensor. Execution of the machine-executable instructions further causes the computational system to repeatedly acquire the temperature data and the optical sensor-specific sensor data as the optical sensor is heated by the heater. The sensor-specific thermal compensation calibration is at least partially determined using the temperature data and the optical sensor-specific sensor data acquired during the heating of the optical sensor. For example, as it is heating, the temperature data and the optical sensor-specific sensor data are acquired repeatedly so that there are a number of temperature points for the calibration. This may provide for a faster calibration as well as over a larger temperature range.

In another example, the at least one light source is a single light source. The multiple light sensors are two light sensors. The optical element comprises two concave surfaces. Each of the two concave surfaces is configured to couple light from the single light source to one of the two light sensors. This may provide for a very accurate and effective way of designing the optical sensor such that it is able to accurately predict the torque and/or force on the drive shaft or cleaning element.

In another example, the single light source is a photo diode or a light-emitting diode.

In another example, the two light sensors are phototransistors.

In another example, the oral cleaning device further comprises a battery for powering the oral cleaning device and a charging system configured for charging the battery. For example the charging system may be configured to inductively couple to an external charging unit and then provide power to charge the battery. The charging system is configured for receiving power from the external charging unit. The oral cleaning device is placeable on the external charging unit. The stationary state detection system comprises the charging system. For example, when power is provided to the charging system and it is detected that it is sitting on or placed on the external charging unit, then this may provide a means of detecting when the oral cleaning device is stationary.

This may be effective because it does not require any additional components to be added to the oral cleaning device to detect when it is stationary. In another example, the oral cleaning device is configured to be mounted on the external charging unit such that the drive shaft is vertical. When the drive shaft is vertical, this may mean that there is zero force or torque on the drive shaft. This may provide for a very beneficial position for determining the sensor-specific thermal compensation calibration.

In another example, the stationary state comprises an accelerometer configured for detecting motion of the oral cleaning device. For example sometimes the oral cleaning device may be set down on a surface when not in use. The use of an accelerometer would be able to detect that the oral cleaning device is not in use and has been set down. Although the drive shaft may not be vertical in this configuration the force or torque on the drive shaft will nonetheless likely be minimal and it may still be possible to acquire data for the sensor-specific thermal compensation calibration.

In another example, the oral cleaning system comprises an example of the oral cleaning device and the external charging unit.

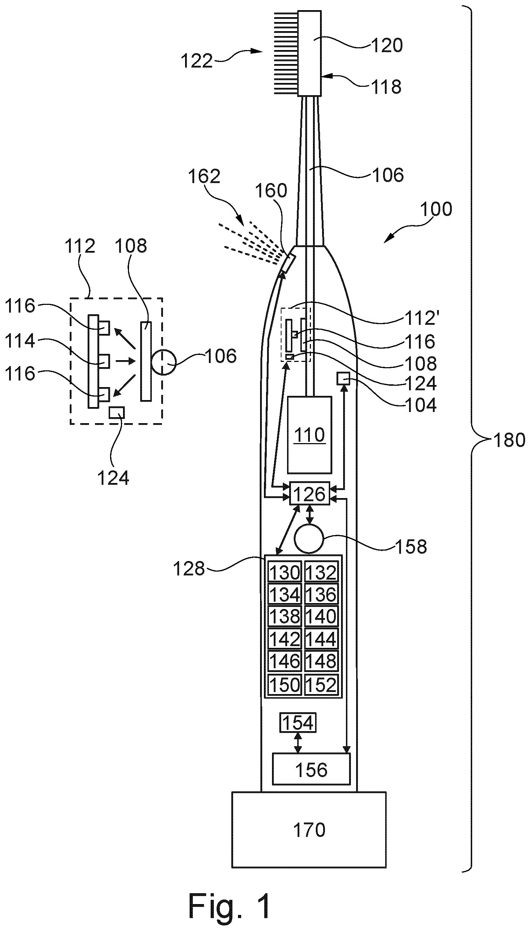

Fig. 1 illustrates an example of an oral cleaning device 100. The oral cleaning device comprises a body portion 102 which forms a handle which an operator can use to hold the oral cleaning device 100. Within the body 102 there is an optional accelerometer 104, which may for example be used to detect if the oral cleaning device is not being used or is in a stationary state.

The oral cleaning device 100 further comprises a drive shaft 106. The drive shaft 106 is connected to a mechanical drive 110 which is used to actuate or move the drive shaft 106. There is a reflective element 108 mounted on the drive shaft 106. The oral cleaning device 100 further comprises an optical sensor 116 which is made up of at least one light source 114 and multiple light sensors 116. The optical sensor is displayed as a top view 112 and as a side view 112'. The reflective element 108 is used to couple light from the light source 114 into the light sensors 116. Changes in the measured amount of light measured by the light sensors 116 are used to measure a deflection or torsion of the mechanical drive 110 and thereby measure a mechanical load on the mechanical drive 110.

Mounted next to or within the optical sensor 116 is a temperature sensor 124 configured for measuring a temperature of the optical sensor 116. The light source 114 and the light sensors 116 may both have a temperature dependence which causes an offset in output of the light sensors 116. The drive shaft 106 is shown as coupling to a cleaning element 118 which in this case is a toothbrush. The cleaning element 118 comprises a brush head 120 with a bristle face 122. Force or torque on the bristle face 122 by an operator will also transmit this torque or force to the drive shaft 106. The optical sensor 112 can measure this torsion or displacement and determine a torque or force from it. The optical sensor 112, 112' may for example be arranged such that when a forces is exerted on the bristle face 122 it causes the distance between the light sensors 116 and the reflective element 108 to increase.

The oral cleaning device 100 is shown as comprising a computational system 126 that is configured for operating and controlling the oral cleaning device 100. It is in communication with the various electronic components of the oral cleaning device 100. The computational system 126 is in communication with a memory 128. The memory 128 is intended to represent various types of memory or data storage accessible to the computational system 126. The memory 128 is shown as containing machine-executable instructions 130. The machine-executable instructions 130 enable the computational system 126 to perform basic numerical and data processing tasks as well as control of the oral cleaning device 100.

The memory 128 is further shown as storing a mechanical load calibration 132. This may for example be a calibration that was determined at the time of manufacture of the oral cleaning device 100. The mechanical load calibration 132 is relatively stable and need only be performed right after manufacture of the oral cleaning device 100. The thermal calibration is more problematic as the exact behavior of the individual light sources 114 and optical sensors 116 can vary greatly from oral cleaning device 100 to oral cleaning device 100. This makes a detailed calibration at the factory difficult.

During the use of the oral cleaning device 100 thermal calibration data 134 may be recorded and then used to calculate a sensor-specific thermal compensation calibration 140. The thermal calibration data 134 could for example be pairs of temperature data 136 and optical sensor-specific sensor data 138 that were acquired at the same time when the oral cleaning device 100 was determined to be in a stationary state.

The oral cleaning device 100 may for example repeatedly or periodically acquire the thermal calibration data 134 and then use this to construct a continually better sensor-specific thermal compensation calibration 140.

Once the sensor-specific thermal compensation calibration 140 has been determined it may be used on the fly or during operation of the oral cleaning device 100 to correct for the offset in the readings from the optical sensor 116. For example, during operation the oral cleaning device 100 may measure a current temperature data 142 and current optical sensor-specific sensor data 144. This may then be used to calculate the offset and calculate corrected optical sensor-specific sensor data 146. In conjunction with the mechanical load calibration 132 an accurate temperature-corrected mechanical load 148 may then be calculated. This may then be compared to a predetermined mechanical load 150. If the predetermined mechanical load 150 is exceeded then a warning signal 152 may be provided.

The oral cleaning device 100 is further shown as comprising an optional battery 154 and optional charging system 156. The optional charging system 156 can for example be used with an external charging unit 170. The external charging unit 170 could function as a base which supports the oral cleaning device in a vertical direction. The charging system 156 could also be used to detect if the oral cleaning device 100 is not being used and is therefore in a stationary state. For example, when the battery 154 is being charged by the charging system 156 then it would be clear that the oral cleaning device 100 would be stationary and mounted to the external charging unit 170.

The oral cleaning device 100 may also have an optional control 158 that enables an operator to turn on/off or operate the oral cleaning device 100 as well as an optional indicator 160 that provides an optical (light) signal 162. The indicator 160 may be used for example to present the warning signal 152. The indicator 160 may for example display different colors of light 162 indicating the amount of force or torque being exerted on the cleaning element 118. For example, green may indicate that both the torque and force are at acceptable levels, yellow may be used to indicate a boundary region where the operator should try to decrease the amount of force or torque, and red may indicate a level of force or torque which may injure or not be conducive for cleaning. The combination of the oral cleaning device 100 and the external charging unit 170 may form an oral cleaning system 180. Although it is not shown in the figure, there may also optionally be a heating element or coil which can be used to controllably heat the optical sensor during calibration.

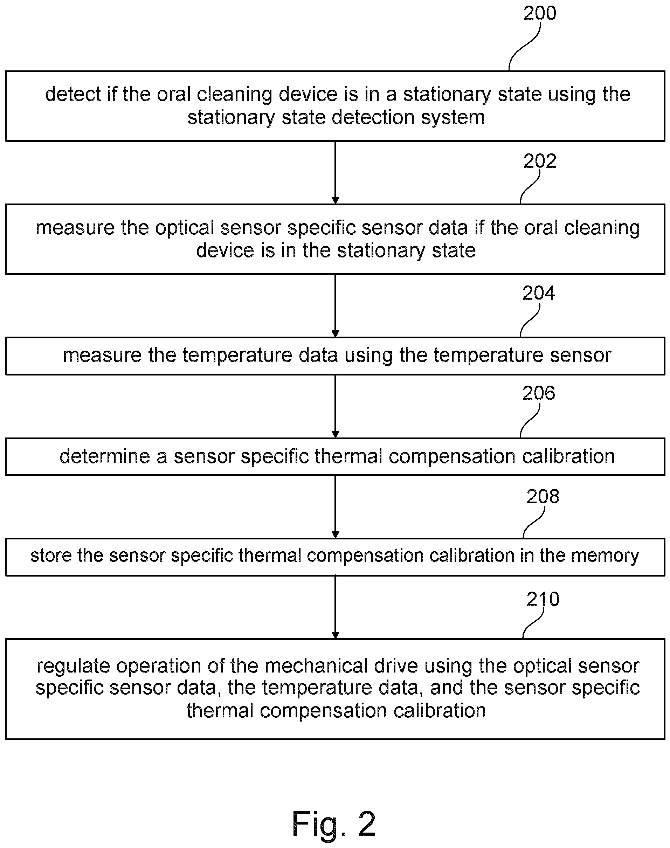

Fig. 2 shows a flowchart which illustrates a method of operating the oral cleaning device. In step 200, it is detected if the oral cleaning device 100 is in a stationary state using the stationary state detection system. For example, using either the optional charging system 156 or the optional accelerometer 104. In step 202, the optical sensor-specific sensor data 138 is measured if the oral cleaning device 100 is in the stationary state. In step 204, the temperature data 136 is measured using the temperature sensor 124. The temperature data is correlated or paired with the optical sensor-specific sensor data 138. In step 206 the sensor-specific thermal compensation calibration 140 is determined. In step 208, the sensor-specific thermal compensation calibration 140 is stored in the memory 128; this may be for further use. Step 210 is optional. In step 210 the operation of the mechanical drive 110 is regulated using the optical sensor-specific sensor data, the temperature data 136 and the sensor-specific thermal compensation calibration 140. For example, this may be used to determine the amount of force or torque on the drive shaft 106 and control the operation of the mechanical drive 110 in response.

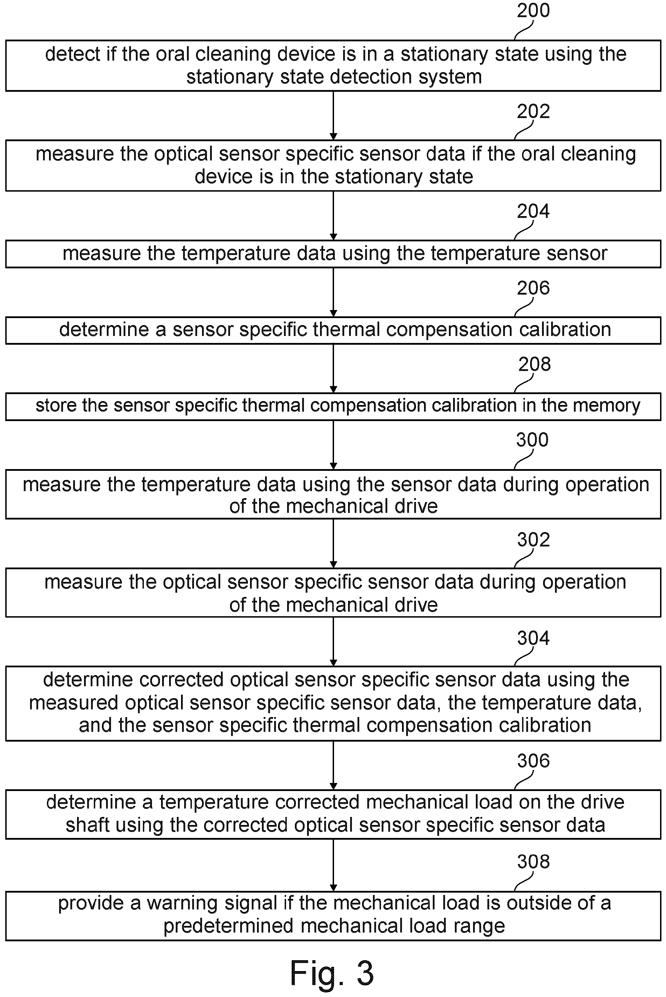

Fig. 3 shows a flowchart which shows an alternative method of operating the oral cleaning device 100. Steps 200, 202, 204, 206, and 208 are performed as was illustrated in Fig. 2. Next, in step 300, the temperature data 142 is measured using the temperature sensor 124 during the operation of the mechanical drive 110. In step 302, the optical sensor-specific sensor data 138 is measured during operation of the mechanical drive 110 also. This current temperature data 142 and the current optical sensor-specific sensor data 144 may be obtained at the same time or within a short interval of each other. In step 304, the corrected optical sensor-specific sensor data 146 is determined using the measured optical sensor-specific sensor data 144, the temperature data 142, and the sensor-specific thermal compensation calibration 140. In step 306, the temperature corrected mechanical load 148 is determined on the drive shaft using the corrected optical sensor-specific sensor data. This may for example be used in conjunction with the mechanical load calibration 132. In step 308, the warning signal 152 is provided if the temperature corrected mechanical load 148 is outside of a predetermined mechanical load range 150.

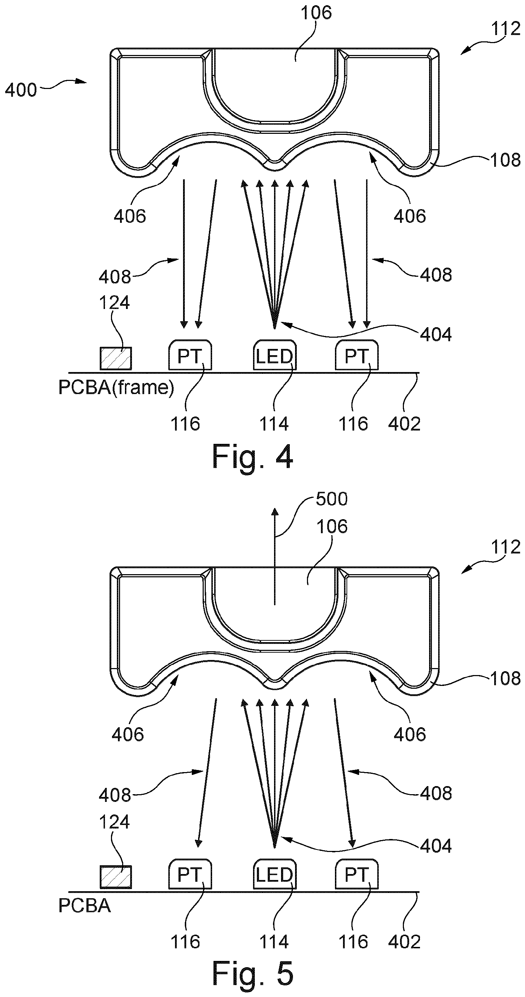

Fig. 4 illustrates an example of the optical sensor 112. There is a reflective element 108 mounted to the drive shaft 106. The sensor 112 is further formed by a light source 114 and two phototransistors 116 mounted on a printed circuit board 402. The light source 114, which in this case is a light-emitting diode, emits light towards the reflective element 108. The reflective element has two concave surfaces 406, each of which individually reflects light back to an individual optical sensor 116. A temperature sensor 124 is also mounted on the circuit board 402 in close proximity to the light source 114 and the optical sensors 116.

Fig. 5 shows the same optical sensor 112 as illustrated in Fig. 4 but now with a brushing force 500 applied. This moves the reflective element 108 away from the circuit board 402 and less reflected light 408 reaches the optical sensors 116. This results in a decrease in light to both optical sensors 116 and may be considered a proportional decrease in the signal from the optical sensors 116.

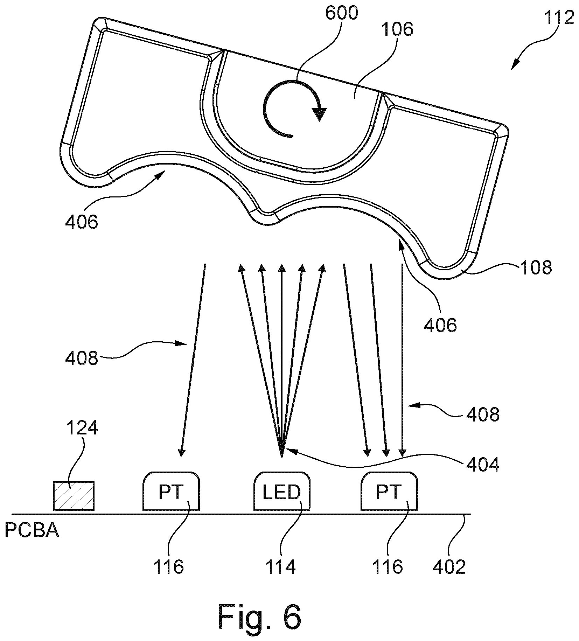

Fig. 6 illustrates the effect of an applied torque 600 to the drive shaft 106. This twists the position of the reflective element 108 and one of the light optical sensors 116 receives less light while the other receives more. This changes a difference in the ratio of the received light or signal between the two optical sensors 112.

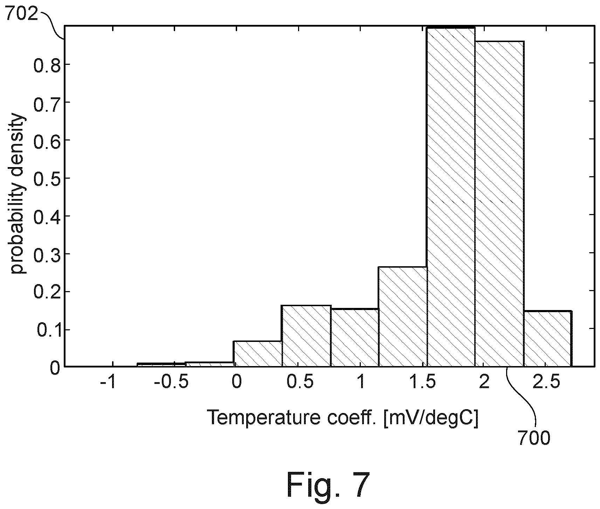

One way of performing the calibration is to assume a linear dependence of the temperature offset of the two optical sensors 116. This can be expressed in the form of a temperature coefficient. The charge in Fig. 7 shows the value of the temperature coefficient 700 for a distribution of oral cleaning devices which have different optical sensors 112 installed to them. From this chart it can be seen that there is a variability between various devices and it is beneficial to perform a thermal calibration of the optical sensor 112.

As a case study, the case where the sensor specific thermal calibration is a sensor specific linear fit is examined. The calibration in this case may be expressed as a temperature coefficient (the slope of the calibration curve). This discussion is relevant e.g. to the cases where only two points are used to determine the calibration or in the case of a linear regression for a larger number of calibration points:

The following exemplary procedure is started when the oral cleaning device 100 (toothbrush) is put on the external charging unit 170 (charger):

The output of the optical sensor 112 is measured and saved in memory ($F_{start}$).

The output of the temperature sensor is measured and saved in the memory ($T_{start}$).

Every n minutes, e.g. every 10 minutes, the appliance will wake up and measure the current temperature ($T_{current}$). If the temperature is higher or lower than the initial temperature ($F_{start}$), the force sensor is started and the measured value is stored in the memory ($F_{current}$).

If the sensor was started in step 3), the temperature coefficient ($c_T$) may be calculated as:

$$c_T = (F_{current} - F_{start})/(T_{current} - T_{start})$$

The procedure above is based on opportunistic learning: if the temperature change is observed, the system will learn / calibrate it's own temperature coefficient.

In a second example forced learning may be used to have a guaranteed temperature increase for learning the temperature coefficient, a heat generating element or heater is used to heat the optical sensor. This can be as simple as a coil that generates heat by forcing DC current through it. The following exemplary procedure may be started when the oral cleaning device 100 (toothbrush) is put on the external charging unit 170 (charger):

The output of the force sensor is measured and saved in memory ($F_{start}$).

The output of the temperature sensor is measured and saved in the memory ($T_{start}$).

The heat generating element is activated for a predetermined duration, for example 30 minutes, on a low power level, such that the sensor will heat up slowly over time.

After 30 minutes, the appliance may wake up and measure the current temperature ($T_{current}$). The force sensor is started, and the measured value is stored in the memory ($F_{current}$).

The temperature coefficient ($c_T$) is calculated as:

$$c_T = (F_{current} - F_{start})/(T_{current} - T_{start})$$

If the temperature coefficient is known, it can used during use of the optical sensor as a compensation factor. If the sensor is activated, the sensor estimate is compensated by the current temperature:

$$F = F_{current} - c_T (T_{current} - T_{reference})$$

Where $c_T$ is the temperature coefficient, $F_{current}$ is the (uncompensated) sensor estimate, $T_{current}$ is the current temperature and $T_{reference}$ is the reference temperature during calibration.

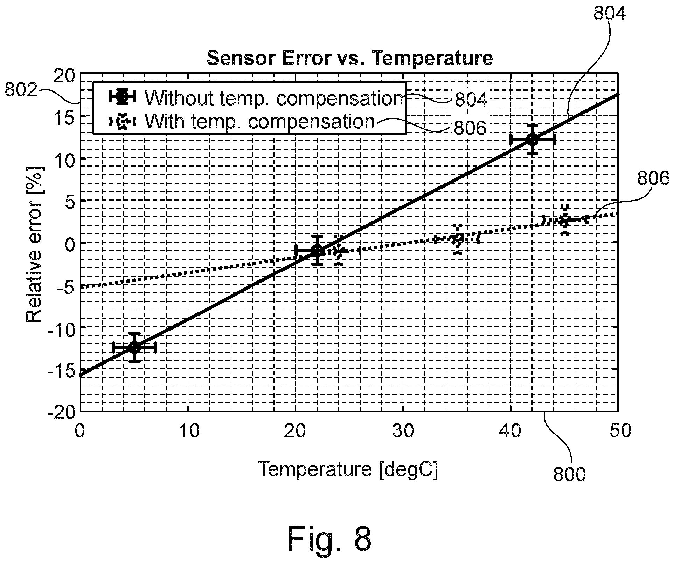

The above case study was tested in an electric toothbrush that incorporated an optical sensor as is depicted in Figs. 4 through 6. The results of the optical sensor are compared with and without temperature compensation, shown in Fig. 8 below.

Fig. 8 further illustrates the benefit of performing examples. In Fig. 8 is a chart of the temperature versus the relative air for the sensor measurements. Curve 804 illustrates the relative air without temperature compensation. Curve 806 represents the relative air using a two-point linear fit to determine the coefficient for a calibration. It can be seen that even using just two data points results in a large increase in the accuracy of the optical sensor 112. The thermal calibration decreased the temperature error of the sensor from 0.66 %/°C to 0.16 %/°C.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bistable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

REFERENCE SIGNS LIST

| | |
|---|---|
| 100 | oral cleaning device |
| 102 | body portion |
| 104 | accelerometer |
| 106 | drive shaft |
| 108 | reflective element |
| 110 | mechanical drive |
| 112 | optical sensor (top view) |
| 112' | optical sensor (side view) |
| 114 | light source |
| 116 | light sensor |
| 118 | cleaning element (tooth brush) |
| 120 | brush head |

| | |
|---|---|
| 122 | bristle face |
| 124 | temperature sensor |
| 126 | computational system |
| 128 | memory |
| 130 | machine executable instructions |
| 132 | mechanical load calibration |
| 134 | thermal calibration data |
| 136 | temperature data |
| 138 | optical sensor specific sensor data |
| 140 | sensor specific thermal compensation calibration |
| 142 | current temperature data |
| 144 | current optical sensor specific sensor data |
| 146 | corrected optical sensor specific sensor data |
| 148 | temperature corrected mechanical load |
| 150 | predetermined mechanical load range |
| 152 | warning signal |
| 154 | battery |
| 156 | charging system |
| 158 | control |
| 160 | indicator |
| 162 | signal |
| 170 | external charging unit |
| 180 | oral cleaning system |
| 200 | detect if the oral cleaning device is in a stationary state using the stationary state detection system |
| 202 | measure the optical sensor specific sensor data if the oral cleaning device is in the stationary state |
| 204 | measure the temperature data using the temperature sensor |
| 206 | determine a sensor specific thermal compensation calibration |
| 208 | store the sensor specific thermal compensation calibration in the memory |
| 210 | regulate operation of the mechanical drive using the optical sensor specific sensor data, the temperature data, and the sensor specific thermal compensation calibration |
| 300 | measure the temperature data using the sensor data during operation of the mechanical drive |
| 302 | measure the optical sensor specific sensor data during operation of the mechanical drive |
| 304 | determine corrected optical sensor specific sensor data using the measured optical sensor specific sensor data, the temperature data, and the sensor specific thermal compensation calibration |
| 306 | determine a temperature corrected mechanical load on the drive shaft using the corrected optical sensor specific sensor data |
| 308 | provide a warning signal if the mechanical load is outside of a predetermined mechanical load range |
| 400 | stationary state |
| 402 | circuit board |
| 404 | emitted light |
| 406 | concave surface |
| 408 | reflected light |
| 500 | applied brushing force |
| 600 | applied torque |
| 700 | temperature coefficient |
| 702 | probability density |
| 800 | temperature |
| 802 | relative error |
| 804 | without temperature compensation |
| 806 | with temperature compensation |

**Claims**

1. An oral cleaning device (100) comprising:

- a body portion (102);
- a stationary state detection system (104, 156);
- a drive shaft (106) configured for receiving a cleaning element (118);
- a reflective element (108) attached to the drive shaft;
- a mechanical drive (110) for mechanically operating the drive shaft;
- an optical sensor (112, 112') configured for measuring a displacement (500, 600) of the drive shaft during operation of the oral cleaning device, wherein the optical sensor comprises at least one light source (114) and multiple light sensors (116), wherein the optical sensor has a fixed position relative to the body portion, wherein the at least one light source is optically coupled to the multiple light sensors via the reflective element, wherein the optical sensor provides optical sensor specific sensor data (138);
- a temperature sensor (124) configured for measuring temperature data descriptive of a temperature of the optical sensor;
- a memory (128) storing machine executable instructions and a mechanical load calibration (132);
- a computational system (126), wherein execution of the machine executable instructions causes the computational system to:

- detect (200) if the oral cleaning device is in a stationary state (400) using the stationary state detection system;
- measure (202) the optical sensor specific sensor data if the oral cleaning device is in the stationary state;
- measure (204) the temperature data, wherein the temperature data is correlated to the optical sensor specific sensor data;
- determine (206) a sensor specific thermal compensation calibration (140); and
- store (208) the sensor specific thermal compensation calibration in the memory.

2. The oral cleaning device of claim 1, wherein execution of the machine executable instructions further causes the computational system to regulate (210) operation of the mechanical drive using the optical sensor specific sensor data, the temperature data, and the sensor specific thermal compensation calibration.

3. The oral cleaning device of claim 1 or 2, wherein execution of the machine executable instructions further causes the computational system to:

   - measure (300) the temperature data (142) using the sensor data during operation of the mechanical drive;
   - measure (302) the optical sensor specific sensor data (144) during operation of the mechanical drive;
   - determine (304) corrected optical sensor specific sensor data (146) using the measured optical sensor specific sensor data, the temperature data, and the sensor specific thermal compensation calibration;
   - determine (306) a temperature corrected mechanical load (148) on the drive shaft using the corrected optical sensor specific sensor data; and
   - provide (308) a warning signal (152, 162) if the temperature corrected mechanical load is outside of a predetermined mechanical load range (150).

4. The oral cleaning device of claim 3, wherein the temperature corrected mechanical load is any one of the following:

   - an external force (500) determined by a reduction in a magnitude of the corrected optical sensor specific sensor data being proportional for the multiple light sensors;
   - an external torque (600) determined by a change in the magnitude of the corrected optical sensor specific sensor data calculated as a ratio between the multiple light sensors; and
   - combinations thereof.

5. The oral cleaning device of claim 3 or 4, wherein the determination of the temperature corrected mechanical load is repeatedly updated during continual operation of the oral cleaning device by repeatedly measuring the temperature data and by repeatedly measuring the optical sensor specific sensor data.

6. The oral cleaning device of any one of the preceding claims, wherein the sensor specific thermal compensation calibration is any one of the following: a sensor specific linear regression calibration, a sensor specific two point linear fit, a sensor specific spline fit, and a sensor specific interpolation between calibration points.

7. The oral cleaning device of any one of the preceding claims, wherein execution of the machine executable instructions further causes the computational system to determine the sensor specific thermal compensation calibration over multiple detections of the oral cleaning device being in the stationary state.

8. The oral cleaning device of claim 6, wherein determining the sensor specific thermal compensation calibration over multiple placements of the oral cleaning device on the external charging unit comprises any one of the following: revising a fit to previously acquired pairs of the temperature data and the optical sensor specific sensor data, replacing the previously acquired pairs of the temperature data and the optical sensor specific sensor data, and averaging with the previously acquired pairs of the temperature data and the optical sensor specific sensor data.

9. The oral cleaning device of any one of the preceding claims, wherein execution of the machine executable instructions further causes the computational system to:

   - calculate projected optical sensor specific sensor data by inputting the temperature data into the sensor specific thermal compensation calibration;
   - perform any one of the following if the optical sensors specific sensor data differs from the projected optical sensor specific sensor data by more than a predetermined amount:

     - provide a calibration warning signal;
     - initiate a thermal compensation calibration recalculation procedure; and
     - combinations thereof.

10. The oral cleaning device of any one of the preceding claims, wherein the oral cleaning device further comprises a heater configured for heating the optical sensor, wherein execution of the machine executable instructions further causes the computational system to:

    - control the heater to heat the optical sensor; and
    - repeatedly acquire the temperature data and the optical sensor specific sensor data as the optical sensor is heated by the heater, wherein the sensor specific thermal compensation calibration is at least partially determined using the temperature data and the optical sensor specific sensor data acquired during the heating of the optical sensor.

11. The oral cleaning device of any one of the preceding claims, wherein the at least one light source is a single light source, wherein the multiple light sensors are two light sensors, wherein the optical element comprises two concave surfaces, wherein each of the two concave surface is configured to couple light from the single light source to one of the two light sensors, wherein the single light source is preferably a photo diode, and wherein the two light sensors are preferably photo transistors.

12. The oral cleaning device of any one of the preceding claims, wherein any one of the following:

- wherein the oral cleaning device further comprises a battery (154) for powering the oral cleaning device and a charging system (156) configured for charging the battery, wherein the charging system is configured for receiving power from an external charging unit (170), when the oral cleaning device is placeable on the external charging unit; wherein the stationary state detection system comprises the charging system, and wherein the oral cleaning device is preferably configured to be mounted on the external charging unit such that the drive shaft is vertical;
- the stationary state comprises an accelerometer (104) configured for detecting motion of the oral cleaning device;
- combinations thereof.

13. An oral cleaning system (180) comprising:

- the oral cleaning device (100) of any one of claim 12, and
- the external charging unit (170).

14. A method of operating an oral cleaning device (100), wherein the oral cleaning device comprises:

- a body portion (102);
- a stationary state detection system (104, 156);
- a drive shaft (106) configured for receiving a cleaning element (118);
- a reflective element (108) attached to the drive shaft;
- a mechanical drive (110) for mechanically operating the drive shaft;
- an optical sensor (112, 112') configured for measuring a displacement (500, 600) of the drive shaft during operation of the oral cleaning device, wherein the optical sensor comprises at least one light source (114) and multiple light sensors (116), wherein the optical sensor has a fixed position relative to the body portion, wherein the at least one light source is optically coupled to the multiple light sensors via the reflective element, wherein the optical sensor provides optical sensor specific sensor data (138);
- a temperature sensor (124) configured for measuring temperature data descriptive of a temperature of the optical sensor; and

wherein the method comprises:

- detecting (200) if the oral cleaning device is in a stationary state (400) using the stationary state detection system;
- measuring (202) the optical sensor specific sensor data if the oral cleaning device is in the stationary state;
- measuring (204) the temperature data, wherein the temperature data is correlated to the optical sensor specific sensor data;
- determining (206) a sensor specific thermal compensation calibration (140); and
- storing (208) the sensor specific thermal compensation calibration.

15. A computer program comprising machine executable instructions (130), wherein the machine executable instructions are configured to cause a computational system (126) to perform the method of claim 14.

Fig. 1

200

detect if the oral cleaning device is in a stationary state using the stationary state detection system

202

measure the optical sensor specific sensor data if the oral cleaning device is in the stationary state

204

measure the temperature data using the temperature sensor

206

determine a sensor specific thermal compensation calibration

208

store the sensor specific thermal compensation calibration in the memory

210

regulate operation of the mechanical drive using the optical sensor specific sensor data, the temperature data, and the sensor specific thermal compensation calibration

Fig. 2

200

detect if the oral cleaning device is in a stationary state using the stationary state detection system

202

measure the optical sensor specific sensor data if the oral cleaning device is in the stationary state

204

measure the temperature data using the temperature sensor

206

determine a sensor specific thermal compensation calibration

208

store the sensor specific thermal compensation calibration in the memory

300

measure the temperature data using the sensor data during operation of the mechanical drive

302

measure the optical sensor specific sensor data during operation of the mechanical drive

304

determine corrected optical sensor specific sensor data using the measured optical sensor specific sensor data, the temperature data, and the sensor specific thermal compensation calibration

306

determine a temperature corrected mechanical load on the drive shaft using the corrected optical sensor specific sensor data

308

provide a warning signal if the mechanical load is outside of a predetermined mechanical load range

Fig. 3

106

112

400

108

406

406

408

408

124

404

PT

LED

PT

PCBA(frame)

116        114        116    402

# Fig. 4

500

106

112

108

406

406

408

408

124

404

PT

LED

PT

PCBA

116        114        116    402

# Fig. 5

Fig. 6

Fig. 7

Fig. 8

Europäisches
Patentamt
European
Patent Office
Office européen
des brevets

## EUROPEAN SEARCH REPORT

Application Number

EP 24 20 3495

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2019/281968 A1 (BOERE STIJN WILLEM [NL] ET AL) 19 September 2019 (2019-09-19) * figures 1-10 * * paragraph [0045] - paragraph [0071] * ----- | 1-15 | INV. A46B15/00 A61B5/00 |
| A | EP 3 092 974 B1 (BRAUN GMBH [DE]) 7 March 2018 (2018-03-07) * paragraph [0016] - paragraph [0024]; figures 1-3 * ----- | 1-15 | |
| A | WO 2017/139256 A1 (ORALUCENT LLC [US]) 17 August 2017 (2017-08-17) * paragraph [0150] - paragraph [0152] * ----- | 1-15 | |
| A | US 5 905 576 A (TAKADA YUJI [JP] ET AL) 18 May 1999 (1999-05-18) * the whole document * ----- | 1-15 | |
| | | | TECHNICAL FIELDS SEARCHED (IPC) |
| | | | A46B A61C A61B |

The present search report has been drawn up for all claims

| Place of search | Date of completion of the search | Examiner |
|---|---|---|
| The Hague | 16 April 2025 | Knoop, Jan |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 20 3495

This annex lists the patent family members relating to the patent documents cited in the above-mentioned European search report.
The members are as contained in the European Patent Office EDP file on
The European Patent Office is in no way liable for these particulars which are merely given for the purpose of information.

16-04-2025

| Patent document cited in search report | | Publication date | Patent family member(s) | | Publication date |
|---|---|---|---|---|---|
| US 2019281968 | A1 | 19-09-2019 | CN | 109152473 A | 04-01-2019 |
| | | | EP | 3462983 A1 | 10-04-2019 |
| | | | JP | 7132852 B2 | 07-09-2022 |
| | | | JP | 2019516448 A | 20-06-2019 |
| | | | RU | 2018145513 A | 25-06-2020 |
| | | | US | 2019281968 A1 | 19-09-2019 |
| | | | WO | 2017202913 A1 | 30-11-2017 |
| EP 3092974 | B1 | 07-03-2018 | CA | 2982713 A1 | 17-11-2016 |
| | | | CN | 107580466 A | 12-01-2018 |
| | | | EP | 3092974 A1 | 16-11-2016 |
| | | | ES | 2670015 T3 | 29-05-2018 |
| | | | JP | 2018514336 A | 07-06-2018 |
| | | | US | 2016331120 A1 | 17-11-2016 |
| | | | WO | 2016181255 A1 | 17-11-2016 |
| WO 2017139256 | A1 | 17-08-2017 | CN | 108697495 A | 23-10-2018 |
| | | | EP | 3413835 A1 | 19-12-2018 |
| | | | WO | 2017139256 A1 | 17-08-2017 |
| US 5905576 | A | 18-05-1999 | DE | 19757716 A1 | 02-07-1998 |
| | | | IT | TO971140 A1 | 24-06-1998 |
| | | | JP | 3799708 B2 | 19-07-2006 |
| | | | JP | H10239046 A | 11-09-1998 |
| | | | KR | 19980064594 A | 07-10-1998 |
| | | | US | 5905576 A | 18-05-1999 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 11051609 B2